# EUROPEAN PATENT SPECIFICATION

(11) **EP 4 288 158 B1**
(45) Date of publication and mention of the grant of the patent: **12.08.2026**
(21) Application number: 22703581.3
(22) Date of filing: 27.01.2022
(51) Int. Cl.: A61Q 17/04, A61K 8/27, A61K 8/29, A61K 8/49

(54) **SUNSCREEN COMPOSITION BASED ON BEMT AND TWO OR MORE PIGMENTARY UV-FILTERS**
SONNENSCHUTZZUSAMMENSETZUNG ENTHALTEND BEMT UND ZWEI ODER MEHR UV-FILTERPIGMENT
COMPOSITION D'ÉCRAN SOLAIRE COMPRENANT DU BEMT AND DES FILTRES UV PIGMENTAIRES

(30) Priority: 03.02.2021 EP 21155060
(43) Date of publication of application: 13.12.2023
(73) Proprietor: DSM IP Assets B.V., 6221 BE Maastricht (NL)
(72) Inventor: JANSSEN, Anne, 4303 Kaiseraugst (CH); KUNZE, Gernot, Ulrich, 4303 Kaiseraugst (CH); RADOMSKY, Karina, 4303 Kaiseraugst (CH); RUDOLPH, Thomas, 4303 Kaiseraugst (CH); VOLLHARDT, Jürgen, Herbert, 4303 Kaiseraugst (CH)
(74) Representative: dsm-firmenich IP
(86) International application number: PCT/EP2022/051911
(87) International publication number: WO 2022/167320

(56) References cited:
- US-A1- 2016 206 527
- US-A1- 2017 151 133
- US-A1- 2018 116 923
- US-A1- 2019 209 631

## Description

The present invention relates to sunscreen compositions containing as UV filters solely a combination of i) bis-ethylhexyloxyphenol methoxyphenyl triazine (BEMT) and (ii) two or more pigmentary UV-filters. Said compositions exhibit an appealing skin feel and look.

There is an increasing need for sunscreens having a sun protection factor (SPF) of 20 or more in order to provide effective protection against UV-radiation. At the same time, however, such sunscreens should still exhibit an appealing skin feel and look and preferably even comply with the GRAS/GRASE requirements of the Federal Food, Drug, and Cosmetic Act.

Pigmentary UV-filters, such as inorganic UV-filters, i.e. micronized (often also called microfine) metal oxides such as titanium dioxide or zinc oxide or micronized insoluble organic UV-filters are particularly useful in sunscreen applications due to their ability to increase the sun protection factor (SPF) of formulations over a broad UV range. In addition, these pigmentary UV-filters are generally regarded as safe for cosmetic use and do not have the disadvantage of a tacky skin-feel as is the case with most soluble organic UV filters. Nevertheless, there are still problems associated with the use of such pigmentary UV-filters. In particular, their efficacy and corelated level of pigmentary powder necessary to achieve proper (and higher) SPF levels makes the product aesthetically and sensorially unacceptable, e.g. providing a heavy and gritty feel and/ or a white/blue residual on the skin.

Even though there has been no lack of attempts to develop efficient sunscreen compositions comprising pigmentary UV-filters with improved sensorial and aesthetic properties such as e.g. a reduced skin whitening effect, this problem has not yet been solved to ultimate satisfaction, particularly in the case of preparations featuring a high sun protection factor.

US 2017/151133 A1 discloses compositions based on microfine coated titanium dioxide and/or microfine coated zinc oxide in combination with uncoated porous silica beads to reduce the whitening effect of said microfine coated titanium dioxide and/or microfine coated zinc oxide.

It is therefore an ongoing need to develop novel sunscreen formulations comprising pigmentary UV-filters which exhibit improved sensorial and/ or aesthetical properties, while having medium or higher sun protection factor (in particular SPF 20 or more).

Surprisingly, it has now be found, that a sunscreen composition comprising bis-ethylhexyloxyphenol methoxyphenyl triazine (BEMT) and two or more pigmentary UV-filters as sole UV-filter substances overcomes the disadvantages of the prior art.

Thus, in a first embodiment, the present invention provides sunscreen compositions containing as UV-filters solely a combination of the UV-filter substances i) bis-ethylhexyloxyphenol methoxyphenyl triazine (BEMT) and (ii) two or more pigmentary UV-filters wherein the two or more pigmentary UV-filters comprise at least two surface coated inorganic UV-filters or at least one surface coated inorganic UV-filter and at least one micronized insoluble organic UV-filter.

Thus, in another embodiment the invention relates to the use of bis-ethylhexyloxyphenol methoxyphenyl triazine to reduce the (skin) whitening effect of two or more pigmentary UV-filters comprised in a sunscreen composition after application to the skin.

In one advantageous embodiments of the present invention the two or more pigmentary UV-filters comprise at least two inorganic UV-filters. Even more preferably at least one titanium dioxide UV-filter and at least one zinc oxide UV-filter are contained in the sunscreen compositions according to the present invention. Most preferably said sunscreen compositions contain as UV-filters solely the combination of i) bis-ethylhexyloxyphenol, (ii-a) a (one) titanium dioxide UV-filter and (ii-b) a (one) zinc oxide UV-filter.

In another advantageous embodiment, the two or more pigmentary UV-filters comprise at least one inorganic and at least one micronized organic UV-filter. Even more preferably at least one titanium dioxide UV-filter and at least micronized methylene bis-benzotriyzolyl tetramethylbutylphenol are contained in the sunscreen compositions according to the present invention. Most preferably in said embodiment, said sunscreen compositions contain as UV-filters solely the combination of i) bis-ethylhexyloxyphenol methoxyphenyl triazine, (ii-a) a (one) titanium dioxide UV-filter and (ii-b) micronized methylene bis-benzotriyzolyl tetramethylbutylphenol.

It is well understood, that in all embodiments of the present invention next to the UV-filters (i) and (ii) no further UV-filters are present in the sunscreen compositions according to the present invention.

Furthermore, it is preferred that the sunscreen compositions according to the present invention do not comprise color pigments and/ or in particular iron color pigments.

Color pigments are well known in the art and used to impart color in particular into decorative cosmetic. Typical pigments include red, brown, russet, black, and yellow iron oxides.

Iron color pigments can either be iron oxides such as in particular α-Fe₂O₃, γ-Fe₂O₃, Fe₃O₄ and FeO, red iron oxide, yellow iron oxide, black iron oxide and brown iron oxide or color pigments comprising iron oxide layers as part of interference color pigments. Examples of such iron color pigments are e.g. listed under the color index numbers CI77499 (Iron Oxides) or CI77491 (Iron Oxides). An example for an effect pigment comprising an iron oxide layers is RonaStar Red Allure (INCI = CI77491 (Iron Oxides), CI77891 (Titanium Dioxide), Silica, Mica).

It is also advantageous in all embodiments of the present invention if the compositions are free of, i.e. do not contain any butyloctyl salicylate (BHB).

The term 'UV-filter' as used herein is well known to a person skilled in the art and refers to any substance which substantially absorbs UV-B and/ or UV-A radiation i.e. with at least one maximum absorbance (peak) between 300 and 400 nm. Such substances are listed under Annex VI of the EU cosmetic product regulation.

Said substantial absorption is in particular characterized by a specific extinction (i.e. E1/1) of at least 130, preferably of at least 150 or even of at least 170, such as of at least 200.

Said specific extinction is well known to a person skilled in the art and refers the absorbance of a 1% solution respectively dispersion (in case of pigmentary UV-filters) of the respective substance in a 1 cm quartz cuvette @ λₘₐₓ.

The term 'pigmentary UV-filter' as used herein refers to UV-filters which are present in the composition in a pigmentary (solid) state Such UV-filters are well known to a person skilled in the art and encompass inorganic UV-filters as well as micronized, insoluble organic UV-filters.

Exemplary UV-filters that are to be absent in the sunscreen composition of the present invention are, with the exception of the bis-ethylhexyloxyphenol methoxyphenyl triazine, any other organic soluble UV-filters such as in particular benzopheone-3, homosalate, ethylhexyl salicylate, ethylhexyl methoxycinnamate, octocrylene, ethylhexyl triazone, butyl methoxydibenzoylmethane and diethylamino hydroxybenzoyl hexyl benzoate
Bis-Ethylhexyloxyphenol methoxyphenyl triazine (also referred to herein as BEMT) is also known as 2,4-bis{[4-(2-ethylhexyloxy)-2-hydroxy]phenyl}-6-(4-methoxyphenyl)-1,3,5-triazine (chemical name) or bemotrizinol (INN) (CAS Number 187393-00-6). BEMT acts as a broad-spectrum UV filter absorbing UVB as well as UVA rays. It has two absorption peaks, 310 and 340 nm. BEMT is suggested for use in sun, day care, alphabetic products such as BB cream and whitening products.

In all embodiments of the present invention, the amount of BEMT in the sunscreen compositions according to the present invention is advantageously selected in the range from 0.4 to 10 wt.-%., preferably in the range from 0.4 to 9 wt.-%, 0.4 to 8 wt.-%, 0.4 to 7 wt.-%, 0.4 to 6 wt.-%, 0.4 to 5 wt.-%, 0.4 to 4 wt.-%, 0.4 to 3 wt.-%, 0.5 to 3 wt.-%, 0.8 to 9 wt.-%, 0.8 to 8 wt.-%, 0.8 to 7 wt.-%, 0.8 to 6 wt.-%, 0.8 to 5 wt.-%, 0.8 to 4 wt.-%, 0.8 to 3 wt.-%, such as for instance in the range from 1 to 5 wt.-%, from 1 to 3 wt.-%, from 2 to 5 wt.-% or from 2 to 4 wt.-%, based on the total weight of the composition.

The term 'inorganic UV-filter' as used herein refers to any metal oxide particles having UV-filter properties as defined above and are thus useful for incorporation into sunscreen compositions as UV filters. Such inorganic UV-filters are well known to a person skilled in the art and are often referred to as micronized or microfine titanium dioxide and zinc oxide.

The particle size of such inorganic UV-filter is not particularly limited. In general, suitable (primary) particle sizes for an efficient UV-light absorption are selected in the range of 2 to 200 nm.

The term 'micronized, insoluble organic UV-filters' as used herein refers to organic UV-filters which are insoluble in the sunscreen composition and which have been micronized. They are characterized by a solubility in water at 25 °C of less than 0.1 wt .-% and a solubility in commonly used cosmetic solvents such as paraffin oil, cyclomethicone, C₁₂-C₁₅ alkyl benzoate, caprylic/ capric triglyceride, coco caprylate/ caprate and dicaprylyl carbonate at 25 °C of less than 1 wt.-%. Said micronized insoluble organic UV-filters have a mean particle size in the range from 0.01 to 2 µm, more preferably from 0.05 to 1.5 µm, especially from 0.08 to 1.0µm such as in particular in the range of 80 to 200 nm.

The insoluble organic UV absorber may be converted into the desired particulate size state by conventional methods, e.g. by grinding the insoluble organic UV absorber, in coarse particle form, in the presence of suitable grinding aids and using known grinding apparatus, e.g., a jet, ball, vibration or hammer mill, preferably a high speed stirring mill or impact mill, especially a rotating ball mill, vibrating mill, tube mill or rod mill.

If not indicated otherwise, the particles sizes as given herein refer to the mean number-based particle size distribution Dn50 (also known as Dn0.5) as determined by laser diffraction e.g. with a Horiba particle size distribution analyzer LA-960 or a Malvern Mastersizer 2000 (ISO 13320:2009).

In all embodiments of the present invention, the (total) amount of the pigmentary UV-filters in the sunscreen composition is preferably selected in the range from 0.5 to 25 wt.-%, more preferably in the range from 1 to 25 wt.-%, and most preferably in the range from 2 to 25 wt.-%, based on the total weight of the composition. Further suitable amounts are selected in the range from 1 to 20 wt.-%, 1 to 15 wt.-%, 1 to 12 wt.-%, 3.0 to 20 wt.-%, 4 to 20 wt.-% and 5 to 20 wt.-%.

In all embodiments of the present invention, the (total) amount of the inorganic UV-filters in the sunscreen compositions according to the present invention is selected in the range of 0.5 to 25 wt.-%, more preferably in the range from 1 to 20 wt.-%, and most preferably in the range from 2 to 15 wt.-%, based on the total weight of the composition. Further suitable amounts are selected in the range from 1 to 15 wt.-%, 2 to 15 wt.-%, 3 to 15 wt.-%, 1 to 10 wt.-%, 2 to 10 wt.-% and 3 to 10 wt.-%. Based on the total weight of the composition.

In all embodiments of the present invention, the (total) amount of the micronized, insoluble organic UV-filters in the sunscreen compositions according to the present invention is selected in the range of 0.5 to 10 wt.-%, preferably in the range of 1 to 10 wt.-%, more preferably in the range of 2 to 10 wt.-%, most preferably in the range of 2 to 6 wt.-% or 3 to 6 wt.-%, based on the total weight of the composition.

Even more advantageously, the (total) amount of inorganic titanium dioxide UV-filters in the sunscreen compositions according to the present invention is selected in the range of 0.5 to 15 wt.-%, preferably in the range of 1 to 15 wt.-%, more preferably in the range of 2 to 10 wt.-%, most preferably in the range of 2 to 6 wt.-% or3 to 6 wt.-%, based on the total weight of the composition.

Even more advantageously, the (total) amount of inorganic zinc oxide UV-filters in the sunscreen compositions according to the present invention is selected in the range of 0.5 to 24.5 wt. %, preferably in the range of 1 to 15 wt.-%, more preferably in the range of 2 to 15 wt.-%, most preferably in the range of 5 to 15 wt.-% or 7.5 to 15 wt.-%, based on the total weight of the composition.

Preferably in all embodiments of the present invention the ratio (w/w) of the titanium dioxide UV-filter(s) to the zinc oxide UV-filter(s) is selected in the range from 1:5 to 5:1, preferably from 1:2 to 2:1. Even more preferably the amount of zinc oxide in the sunscreen compositions according to the present invention is higher than the amount of titanium dioxide such as in the range of 5:1 to 1.25:1, most preferably in the range of 2.5:1 to 1.25:1.

According to the invention, the two or more pigmentary UV-filters comprise at least two surface coated inorganic UV-filters or the two or more pigmentary UV-filters comprise at least one surface coated inorganic UV-filter and at least one micronized insoluble organic UV-filter.

The surface coating may comprise providing the metal oxide particles with a thin hydrophilic or hydrophobic inorganic or organic layer by methods known per se. According to the present invention the different surface coatings can also comprise water. As a result of the surface treatment, the metal oxide is given a hydrophilic, amphiphilic or hydrophobic character.

Examples of inorganic surface coatings which are suitable for the purposes of the instant invention comprise aluminum oxide (Al₂0₃), aluminum hydroxide Al(OH)₃, aluminum oxide hydrate (also: Alumina, CAS-No.: 1333-84-2), sodium hexametaphosphate (NaPO₃)₆, sodium meta-phosphate (NaPO₃)ₙ, silicon dioxide (SiO₂) (also: Silica, CAS-No.: 7631-86-9), and iron oxide (Fe₂O₃). These inorganic surface coatings can be present on their own, in combination and/or in combination with organic coating materials, in particular as outlined below.

Examples of organic surface coatings which are suitable for use in the present invention include vegetable or animal aluminum stearate, fatty acids such as stearic acid and lauric acid, dimethylpolysiloxane (also: dimethicone), methylpolysiloxane (methicone), simethicone, triethoxycaprylylsilane, octyltrimethoxysilane and cetyl phosphates such as potassium cetyl phosphate as well as any mixtures thereof. These organic surface coatings can be present on their own, in combination and/or in combination with inorganic coating materials.

The crystalline form of the titanium dioxide may be of any crystal or amorphous type. For example, titanium dioxide may be any type of amorphous, rutil, anastase, brookite or a mixture thereof. Preferably, in all embodiments of the present invention, the crystalline form of the titanium dioxide is rutil.

It is furthermore preferred that the zinc oxide is a white powder consisting of zinc oxide present as wurtzite crystal structures.

Titanium dioxide UV-filters for use according to the present invention including any pre-dispersions thereof are e.g. available as PARSOL^{®} TX (INCI: titanium dioxide, silica, dimethicone) at DSM Nutritional Products Ltd., Micro Titanium dioxide MT-01 at Tayca or TTO-55I at Ishihara Sangyo Kaisha. Also suitable is double coated titanium dioxide having an inner alumina coating and an outer simethicone coating e.g. commercially available as Eusolex T-2000 at EMD chemicals Inc./Rona.

Zinc oxide UV-filters for use according to the present invention including any pre-dispersions thereof are e.g. available from BASF as Z-Cote or Z-Cote HP1 (2% dimethicone coating), from Tayca as MZ-505S (5% methicone coating), or from DSM Nutritional Products Ltd as PARSOL^{®} ZX (2-3.5% triethoxycaprylylsilane coating).

In a particular embodiment the sunscreen compositions according to the present invention comprise as inorganic UV-filters at least one double coated titanium dioxide and at least one coated zinc oxide, most preferably in the absence of any further microfine metal oxides (i.e. one double coated titanium dioxide and one coated zinc oxide).

Most preferably in all embodiments according to the present invention the titanium dioxide UV-filter is either a double coated titanium dioxide, even more preferably a double coated titanium dioxide having an inner silica and an outer organic coating or a silica coated titanium dioxide.

Preferably, the inner silica coating layer of the double coated titanium dioxide consists of a minimum of 0.5 wt.-% of inorganic silica (based on titanium dioxide). More, preferably the inner coating layer consists of 0.5 wt.-% to 50 wt.-%, most preferably of 1 wt.-% to 20 wt.-% of inorganic silica (based on titanium dioxide).

The outer coating of the double coated titanium dioxide is preferably selected from the class of organic coatings such e.g. silicone oils (e.g. simethicones, methicones, dimethicones, polysilicone-15), alkyl silanes (e.g. octyl tri(m)ethoxy silane), olefinic acids (e.g. stearic acid), or polyols (e.g. glycerol) and can be applied to the titanium dioxide particle by methods known to a person skilled in the art e.g. described in FI57124. Preferably the outer coating is selected from the group consisting of simethicone, methicone, dimethicone, polysilicones-15, stearic acid and octyl trimethoxy silane. Most preferably the outer coating is dimethicone. Even more preferably, the amount of the outer coating layer is at least 0.25 wt.-% based on the titanium dioxide. Preferably the amount of the outer coating is selected in the range of 0.5 wt.-% to 50 wt.-%, most preferably in the range of 0.5 wt.-% to 10 wt.-%, based on the titanium dioxide.

In all embodiments of the present invention, the double coated titanium dioxide according to the present invention preferably has a titanium dioxide content selected in the range of 70-95 wt.-% and a silicon dioxide content selected in the range of 5-20 wt.-%, such as preferable a titanium dioxide content selected in the range of 80-90 wt.-% and a silicon dioxide content selected in the range of 10 to 15 wt.-%, with the proviso that the total content of titanium dioxide and silicone dioxide is selected in the range of 90 - 100 wt.-%.

Preferably, the double coated titanium dioxide has a mean primary particle size in the range from 2 to 100 nm, more preferably in the range of 5 to 50 nm, most preferably in the range of 10 to 25 nm and a secondary particle size between 0.025 and 1 µm, such as preferably between 0.05 and 0.075 µm.

Particularly suitable double coated titanium dioxide according to the present invention contains a rutil-type titanium dioxide (TiO₂) core with a double coating of silica (inner coating) and dimethicone (outer coating) and has titanium dioxide content of at least 75 wt.-%, preferably in the range from 82-87 wt.-% and a silicon dioxide content of at least 10 wt.-%, preferably in the range from 10.5 to 14.5 wt.-%, and a mean particle size distribution Dₙ50 of 25 to 100 nm, preferably 40 to 80 nm (analysed by Laser diffraction measurements with a Malvern Mastersizer 2000), which double coated titanium dioxide is e.g. commercially available as PARSOL^{®} TX (INCI: titanium dioxide, silica, dimethicone) at DSM nutritional products Ltd.

Most preferably, in all embodiments according to the present invention, the zinc oxide is either uncoated or coated with triethoxycaprylylsilane. Most preferably, the zinc oxide is a white powder consisting of zinc oxide present as wurtzite crystal structures, coated with triethoxycaprylylsilane, which has a zinc oxide content of 96-98%, a triethoxycaprylylsilane content of 2-3.5 % and a mean particle size of 90 to 130 nm (analysed by Laser diffraction measurements with a Malvern Mastersizer 2000) which is commercially available as PARSOL^{®} ZX from DSM Nutritional Products Ltd.

Preferred micronized, insoluble organic UV-filters encompass micronized methylene bis-benzotriazolyl tetramethylbutylphenol and micronized tris-biphenyl triazine. Most preferred in all embodiments of the present invention is the use of micronized methylene bis-benzotriazolyl tetramethylbutylphenol. Said UV-filters are commercially available as aqueous dispersions thereof under the tradename PARSOL^{®} MAX from DSM Nutritional Products or Tinosorb^{®} A2B from BASF. Next to the respective micronized UV-filter in an amount of 40 to 60 wt.-%, said dispersions consist essentially of 5 to 10 wt.-% of decylglucoside, 0.1 to 1 wt.-% of a thickener such as xanthan gum and 0 to 1.5 wt.-% of an additive such as in particular selected from the group of propylene glycol, butylene glycol, disodium phosphate and/ or a silicon defoamer such as simethicone. Said micronized, insoluble organic UV-filters exhibit a mean particle size d(0.5) as e.g. outlined in the opinion of the Scientific Committee on Consumer Safety (SCCS) on 2,2'-Methylene-bis-(6-(2H-benzotriazol-2-yl)-4-(1,1,3,3-tetramethylbutyl)phenol) and on 1,3,5-Triazine, 2,4,6-tris[1,1'-biphenyl]-4-y in the range of about 100 to about 150 nm (mass based) as measured by the FOQELS technique.

Particularly suitable dispersions of methylene bis-benzotriazolyl tetramethylbutylphenol (MBBT) essentially consist of 45 to 55 wt.-% MBBT, 6.0 to 10.0 wt.-% decyl glucoside, 0.2 to 0.6 wt.-% propylene glycol, 0.1 to 0.5 wt.-% xanthan gum and water (ad 100 wt.-%).

Particularly suitable dispersions of tris-biphenyl triazine (TBPT) essentially consist of 47 to 53 wt.-% TBPT, 6.5 to 8.5 wt.-% decyl glucoside, 0.2 to 0.6 wt.-% disodium phosphate, 0.2 to 0.6 wt.-% butylene glycol, 0.1 to 0.3 wt.-% xanthan gum and water (ad 100 wt.-%).

Particularly advantageous combinations of pigmentary UV-filters (ii) according to the present invention encompass the combination of a double coated titanium dioxide having an inner silica and an outer dimethicone coating and a zinc oxide coated with triethoxycaprylylsilane, the combination of a silica coated titanium dioxide and an uncoated zinc oxide as well as the combination of a double coated titanium dioxide having an inner silica and an outer dimethicone coating and the micronized, insoluble organic UV-filter methylene bis-benzotriazolyl tetramethylbutylphenol.

As the sunscreen compositions according to the invention are intended for topical application, it is well understood that they comprise a physiologically acceptable medium, i.e. a medium compatible with keratinous substances, such as the skin, mucous membranes, and keratinous fibres. In particular, the physiologically acceptable medium is a cosmetically acceptable carrier.

The term 'cosmetically acceptable carrier' as used herein refers to all carriers and/or excipients and/ or diluents conventionally used in topical cosmetic compositions such as in particular in skin care preparations.

The exact amount of carrier will depend upon the actual level of the UV filters and any other optional ingredients that one of ordinary skill in the art would classify as distinct from the carrier (e.g., other active ingredients).

In an advantageous embodiment, the sunscreen compositions according to the present invention comprise from 50% to 99%, preferably from 60% to 98%, more preferably from 70% to 98%, such as in particular from 80% to 95% of a carrier, based on the total weight of the sunscreen composition.

In a particular advantageous embodiment, the carrier consists furthermore of at least 30 wt. %, more preferably of at least 40 wt.-%, most preferably of at least 45 wt.-% of water, such as in particular of 50 to 90 wt.-% of water.

In particular, the sunscreen compositions according to the present invention are cosmetic or pharmaceutical compositions, preferably cosmetic (non-therapeutic) compositions.

In one embodiment, the sunscreen compositions according to the present invention are applied to mammalian keratinous tissue such as in particular to human skin or the human scalp and hair.

The term "cosmetic composition" as used in the present application refers to cosmetic compositions as defined under the heading "Kosmetika" in Römpp Lexikon Chemie, 10^{th} edition 1997, Georg Thieme Verlag Stuttgart, New York as well as to cosmetic compositions as disclosed in A. Domsch, "Cosmetic Compositions", Verlag für chemische Industrie (ed. H. Ziolkowsky), 4^{th} edition, 1992.

Preferred sunscreen compositions according to the invention are skin care preparations, decorative preparations, and functional preparations.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of decorative preparations are, in particular, lipsticks, eye shadows, mascaras, dry and moist make-up formulations, rouges and/or powders.

Examples of functional preparations are cosmetic or pharmaceutical compositions containing active ingredients such as hormone preparations, vitamin preparations, vegetable extract preparations, anti-ageing preparations, and/or antimicrobial (antibacterial or antifungal) preparations without being limited thereto.

In a particular embodiment, the sunscreen compositions according to the invention are light-protective preparations (sun care products, sunscreens), such as sun protection milks, sun protection lotions, sun protection creams, sun protection oils, sun blocks or tropical's or day care creams with a SPF (sun protection factor). Of particular interest are sun protection creams, sun protection lotions and sun protection milks.

The compositions of the invention (including the carrier) may comprise conventional adjuvants and additives, such as preservatives/antioxidants, fatty substances/oils, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing agents, as well as agents to improve elasticity and skin barrier and carriers and/or excipients or diluents conventionally used in sunscreen compositions.

If nothing else is stated, the excipients, additives, diluents, etc. mentioned in the following are suitable for sunscreen compositions according to the present invention. The necessary amounts of the cosmetic and dermatological adjuvants and additives can, based on the desired product, easily be determined by the skilled person.

The additional ingredients can either be added to the oily phase, the aqueous phase or separately as deemed appropriate. The mode of addition can easily be adapted by a person skilled in the art.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

Preferred sunscreen compositions in all embodiments of the present invention are emulsions containing an oily phase and an aqueous phase such as in particular an O/W, W/O, Si/W, W/Si, O/W/O, W/O/W multiple or a pickering emulsions. The amount of the oily phase (i.e. the phase containing all oils and fats) present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the composition.

According to one even more preferred embodiment, the sunscreen compositions according to the present invention as outlined herein are O/W emulsions comprising an oily phase dispersed in an aqueous phase in the presence of an O/W emulsifier. The preparation of such O/W emulsions is well known to a person skilled in the art and illustrated in the examples.

In one advantageous embodiment, the O/W emulsifier is a phosphate ester emulsifier. Among the preferred phosphate ester emulsifier are C8-10 Alkyl Ethyl Phosphate, C9-15 Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C12-15 Pareth-2 Phosphate, C12-15 Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate. A particular phosphate ester emulsifier according to the invention is potassium cetyl phosphate e.g. commercially available as Amphisol^{®} K at DSM Nutritional Products Ltd Kaiseraugst.

Further suitable O/W emulsifiers according to the present invention encompass PEG 30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, glycerylstearatcitrate, glycerylstearate (self-emulsifying), stearic acid, salts of stearic acid, polyglyceryl-3-methylglycosedistearate. Further suitable emulsifiers are sorbitan oleate, sorbitan sesquioleate, sorbitan isostearate, sorbitan trioleate, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C10-30 alkyl acrylate crosspolymer, acrylates/steareth-20 methacrylate copolymer, PEG-22/dodecyl glycol copolymer, PEG-45/dodecyl glycol copolymer, and mixtures thereof.

Another particular suitable class of O/W emulsifier are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

Further suitable are commercially available polymeric emulsifiers such as hydrophobically modified polyacrylic acid such as Acrylates/C10-30 Alkyl Acrylate Crosspolymers which are commercially available under the tradename Pemulen^{®} TR-1 and TR-2 by Noveon.

Another class of particularly suitable emulsifiers are polyglycerol esters or diesters of fatty acids also called polyglyceryl ester/ diester (i.e. a polymer in which fatty acid(s) is/ are bound by esterification with polyglycerine), such as e.g. commercially available at Evonik as Isolan GPS [INCI Name Polyglyceryl-4 Diisostearate/Polyhydroxystearate/Sebacate (i.e. diester of a mixture of isostearic, polyhydroxystearic and sebacic acids with Polyglycerin-4)] or Dehymuls PGPH available at Cognis (INCI Polyglyceryl-2 Dipolyhydroxystearate).

Also suitable are polyalkylenglycolether such as Brij 72 (Polyoxyethylen(2)stearylether) or Brij 721 (Polyoxyethylene (21) Stearyl Ether e.g. available at Croda.

Particularly advantageous O/W emulsifiers according to the present invention are one or more of Polyglyceryl-3 Methylglucose Distearate, Lauryl Glucoside (and) Polyglyceryl-2 Dipolyhydroxystearate, Glyceryl Sterate Citrate, Sodium Cetearyl Sulfate, Cetearyl Glucoside; Polyglyceryl-6 Stearate (and) Polyglyceryl-6 Behenate, Cetearyl Olivate (and) Sorbitan Olivate, Arachidyl Alcohol (and) Behenyl Alcohol (and) Arachidyl Glucosides, Cetearyl Alcohol (and) Coco-Glucoside, Coco-Glucoside (and) Coconut Alcohol, PEG-100 Stearate (and) Glyceryl Stearate, Sodium Stearoyl Glutamate, Steareth-20, Steareth-21, Steareth-25, Steareth-2, Ceteareth-25 and Ceteareth-6 (all listed by their INCI names).

It is particularly preferred in accordance with the invention if the composition comprises potassium cetyl phosphate as emulsifier.

The at least one O/W respectively Si/W emulsifier is preferably used in an amount of 0.5 to 10 wt. % such as in particular in the range of 0.5 to 5 wt.-% such as most in particular in the range of 0.5 to 4 wt.-%, based on the total weight of the composition.

Suitable W/O- or W/Si-emulsifiers according to the present invention are polyglyceryl-2-dipolyhydroxystearat, PEG-30 dipolyhydroxystearat, cetyl dimethicone copolyol, polyglyceryl-3 diisostearate polyglycerol esters of oleic/isostearic acid, polyglyceryl-6 hexaricinolate, polyglyceryl-4-oleate, hexadecaneol-4 oleate/PEG-8 propylene glycol cocoate, magnesium stearate, sodium stearate, potassium laurate, potassium ricinoleate, sodium cocoate, sodium tallowate, potassium castorate, sodium oleate, and mixtures thereof. Further suitable W/Si-emulsifiers are Lauryl Polyglyceryl-3 Polydimethylsiloxyethyl Dimethicone and/or PEG-9 Polydimethylsiloxyethyl Dimethicone and/or Cetyl PEG/PPG-10/1 Dimethicone and/or PEG-12 Dimethicone Crosspolymer and/or PEG/PPG-18/18 Dimethicone. The at least one W/O emulsifier is preferably used in an amount of about 0.001 to 10 wt.-%, more preferably in an amount of 0.2 to 7 wt.-% with respect to the total weigh of the composition.

The sunscreen compositions according to the present invention furthermore advantageously contain at least one co-surfactant such as e.g. selected from the group of mono- and diglycerides and/ or fatty alcohols. The co-surfactant is generally used in an amount selected in the range of 0.1 to 10 wt.-%, such as in particular in the range of 0.5 to 7 wt.-%, such as most in particular in the range of 1 to 5 wt.-%, based on the total weight of the composition. Particular suitable co-surfactants are selected from the list of alkyl alcohols such as cetyl alcohol (Lorol C16, Lanette 16), cetearyl alcohol (Lanette O), stearyl alcohol (Lanette 18), behenyl alcohol (Lanette 22), polyhydroxystearic acid, glyceryl stearate, glyceryl myristate (Estol 3650), hydrogenated coco-glycerides (Lipocire Na10) as well as mixtures thereof.

In all embodiments of the present invention, it is particular advantageous if the composition also comprises cetyl alcohol, stearyl alcohol and/or glycerylstearate, preferably stearyl alcohol.

It is particular advantageous, if the topical compositions of the present invention further comprise polyhydroxystearic acid as well as mixtures thereof.

Please check the following part in particular also in view of adding other ingredients which should be covered and could be used to generate (i) market relevant novelty and/ or inventive step.

Advantageous embodiments of the composition of the present invention also include those wherein the composition comprises one or more oils selected from butylene glycol dicaprylate/dicaprate, phenethyl benzoate, C₁₂-C₁₅ alkyl benzoate, dibutyl adipate, diisopropyl sebacates, dicaprylyl carbonate, di-C₁₂-₁₃ alkyl tartrates, diethylhexyl syringylidene malonates, hydrogenated castor oil dimerates, triheptanoin, C₁₂-₁₃ alkyl lactates, C₁₆₋₁₇ alkyl benzoates, propylheptyl caprylates, caprylic/capric triglycerides, diethylhexyl 2,6-naphthalates, octyldodecanol, ethylhexyl cocoates. Preferably, the composition according to the present invention comprises as oil(s) butylene glycol, dibutyl adipate, phenethyl benzoate, dicaprylyl carbonate, C₁₂-C₁₅ alkylbenzoate, caprylyl carbonate, capric/caprylic triglyceride as well as mixtures thereof, most preferably butylene glycol, phenethyl benzoate and C₁₂-C₁₅ alkylbenzoate.

In a still further advantageous aspect of the invention, the sunscreen compositions of the present invention further comprise a preservative and/ or a preservative booster, preferably selected from the group consisting of ethanol, phenoxyethanol, ethylhexylglycerin, hexylglycerin, glyceryl caprylate, 1,2-pentanediol, 1,2-hexanediol, 1,2-octanediol (caprylyl glycol), 1,2-decanediol and 2-methyl-1,3-propanediol as well as mixtures thereof, most preferably selected from the group of phenoxyethanol and ethylhexylglycerin as well as mixtures thereof. When present, the preservative respectively the preservative booster is preferably used in an amount of 0.01 to 2 wt. %, more preferably in an amount of 0.05 to 1.5 wt.-%, most preferably in an amount of 0.1 to 1.0 wt.-%, based on the total weight of the composition.

It is advantageous in accordance with the invention if the preparation comprises one or more alkanediols from the group 1,2-pentanediol, 1,2-hexanediol, 1,2-octanediol, 1,2-decanediol and 2-methyl-1,3-propanediol. When present, the one or more alkanediols is preferably used in an amount of 0.1 to 7 wt. %, more preferably in an amount of 0.5 to 7 wt.-%, most preferably in an amount of 1.0 to 5.0 wt.-%, based on the total weight of the composition.

It is further advantageous in accordance with the invention if the composition of the invention comprises ethanol, phenoxyethanol and/or ethylhexylglycerin.

In another advantageous aspect, the sunscreen compositions according to the present invention are free of any parabenes, benzethoniumchlorid, piroctone olamine, lauroylarginat, methylisothiazolinon, chlormethylisothiazolinon, bronopol, benzalkoniumchloride, formaldehyd releasing compounds, salicylic acid, triclosan, DMDM hydantoin, chlorphenesin and IPBC (lodopropinylbutyl carbamate).

The sunscreen compositions according to the invention may further contain one or more emollients which soothe and soften the skin. As an example, the emollient may be silicone (dimethicone, cyclomethicone), vegetable oils (grape seed, sesame seed, jojoba, etc.), butters (cocoa butter, shea butter), and petrolatum derivatives (petroleum jelly, mineral oil).

In another aspect, the sunscreen composition of the invention may comprise one or more fragrances selected from limonene, citral, linalool, alpha-isomethylionone, geraniol, citronellol, 2-isobutyl-4-hydroxy-4-methyltetrahydropyran, 2-tert-pentylcyclohexyl acetate, 3-methyl-5-phenyl-1-pentanol, 7-acetyl-1,1,3,4,4,6-hexamethyltetralin, adipic diester, cinnamal, amyl salicylate, alpha-amylcinnamaldehyde, alpha-methylionone, butylphenylmethylpropional, cinnamal, amylcinnamyl alcohol, anise alcohol, benzoin, benzyl alcohol, benzyl benzoate, benzyl cinnamate, benzyl salicylate, bergamot oil, bitter orange oil, butylphenylmethylpropional, cardamom oil, cedrol, cinnamal, cinnamyl alcohol, citronellyl methylcrotonate, citrus oil, coumarin, diethyl succinate, ethyllinalool, eugenol, Evernia furfuracea extract, Evernia prunastri extract, farnesol, guaiacwood oil, hexylcinnamal, hexyl salicylate, hydroxycitronellal, lavender oil, lemon oil, linalyl acetate, mandarin oil, menthyl PCA, methyl hexadecan, nutmeg oil, rosemary oil, sweet orange oil, terpineol, tonkabean oil, triethyl citrate, vanillin.

The composition of the invention may advantageously comprise moisturizers. Moisturizers are compounds or mixtures of compounds which give cosmetic compositions the quality, after application to or distribution on the skin surface, of reducing the loss of moisture of the stratum corneum (also called transepidermal water loss (TEWL)) and/or of positively influencing the hydration of the stratum corneum.

Non-limiting examples of advantageous moisturizers for use in the present invention include glycerol, lactic acid and/or lactates, especially sodium lactate, butylene glycol, propylene glycol, biosaccharide gum-1, Glycine soya, ethylhexyloxyglycerol, pyrrolidonecarboxylic acid, and urea. Of further advantage, in particular, is the use of polymeric moisturizers from the group of the polysaccharides which are water-soluble and/or swellable in water and/or gellable with the aid of water. Especially advantageous, for example, are hyaluronic acid, chitosan and/or a fucose-rich polysaccharide which is registered in Chemical Abstracts under the registry number 178463-23-5 and is available, for example, under the Fucogel^{®}1000 name from the company SOLABIA S.A. Moisturizers may also be used advantageously as active antiwrinkle ingredients for protection from changes to the skin of the kind occurring in skin aging, for example.

The cosmetic compositions of the invention may further comprise advantageously, although not mandatorily, fillers which have the effect, for example, of further improving the sensorial and cosmetic properties of the formulations and evoking or intensifying a velvety or silky skin sensation, for example. Advantageous fillers in the sense of the present invention are starch and starch derivatives (such as tapioca starch, distarch phosphate, aluminum or sodium starch octenylsuccinate, and the like, for example), Valvance pigments which have neither primarily UV filter effect nor coloring effect (such as Valvance Touch 210 or 250 for example) and/or Aerosils^{®} (CAS No. 7631-86-9) and/or talc and/or polyethylene, nylon, and silica dimethyl silylate.

The water phase of the compositions of the invention may advantageously comprise customary cosmetic auxiliaries, such as, for example, alcohols, particularly those of low C number, preferably ethanol and/or isopropanol, or polyols of low C number, and also ethers thereof, preferably propylene glycol, glycerol, electrolytes, self-tanning agents, and also, in particular, one or more thickeners, which may be advantageously selected from the group of silicon dioxide, aluminum silicates, polysaccharides and/or derivatives thereof, e.g., hyaluronic acid, xanthan gum, hydroxypropylmethylcellulose, particularly advantageously from the group of polyacrylates, preferably a polyacrylate from the group referred to as Carbopols, examples being carbopols of types 980, 981, 1382, 2984, and 5984, in each case individually or in combination. Further thickeners advantageous in accordance with the invention are those having the INCI designation Acrylates/C10-30 Alkyl Acrylate Crosspolymer (e.g., Pemulen TR 1, Pemulen TR 2, Carbopol 1328 from NOVEON) and also Aristoflex AVC (INCI: Ammonium Acryloyldimethyltaurate/VP Copolymer) as well as Simugel NS (INCI: Hydroxyethyl Acrylate/Sodium Acryloyldimethyl Taurate Copolymer & Squalane & Polysorbate 60).

It is preferred in accordance with the invention if the composition comprises xanthan gum, crosslinked acrylate/C10-C30 alkyl acrylate polymer, hydroxyethyl acrylate/sodium acryloyldimethyl taurate copolymer, and/or vinylpyrrolidone/hexadecane copolymer, preferably xanthan gum and/ or hydroxyethyl acrylate/sodium acryloyldimethyl taurate.

The sunscreen compositions of the invention manage with a surprisingly small total amount of UV filters.

In another aspect, the composition may have an SPF of at least 20, preferably of at least 30.

Advantageously in accordance with the invention, the composition of the invention comprises film formers. Film formers in the sense of the present invention are substances of various constitutions and are characterized by the following properties: When a film former is dissolved in water or other suitable solvents, and when the solution is then applied to the skin, the film former, following evaporation of the solvent, forms a film which serves essentially to fix the UV-filters on the skin and thus to increase the water resistance of the product.

It is especially advantageous to select the film formers from the group of the polymers based on polyvinylpyrrolidone (PVP)
Particular preference is given to copolymers of vinylpyrrolidone, as for example the PVP hexadecane copolymer and the PVP eicosene copolymer, which are available under the trade names Antaron V216 and Antaron V220 from GAF Chemicals Corporation.

Likewise advantageous are further polymeric film formers, such as, for example, sodium polystyrene sulfonate, which is available under the trade name Flexan 130 from National Starch and Chemical Corp., and/or polyisobutene, available from Rewo under the trade name Rewopal PIB1000. Examples of further suitable polymers are polyacrylamides (Seppigel 305), polyvinyl alcohols, PVP, PVP/VA copolymers, polyglycols, acrylate/octylacrylamide copolymer (Dermacryl 79) Likewise advantageous is the use of hydrogenated castor oil dimer dilinoleate (CAS 646054-62-8, INCI Hydrogenated Castor Oil Dimer Dilinoleate), which can be acquired from Kokyu Alcohol Kogyo under the name Risocast DA-H, or else PPG-3 benzyl ether myristate (CAS 403517-45-3), which can be acquired under trade name Crodamol STS from Croda Chemicals.

The sunscreen compositions according to the invention in general have a pH in the range of 3 to 10, preferably a pH in the range of 4 to 8 and most preferably a pH in the range of 4 to 7. The pH can easily be adjusted as desired with suitable acids such as e.g. citric acid or bases such as NaOH according to standard methods in the art.

The following examples are provided to further illustrate the compositions and effects of the present invention.

### Experimental Part

The formulations (O/W emulsions) as outlined in Table 1 have been prepared according to standard methods in the art. Then the whitening on LENETA black cards has been tested according to the method as outlined below:
- Apply 300mg of cream on LENETA black card (3 cards are used for each formulation)
- cream is spread by a spreading device (speed: 10mm/s) using a 60µm spreading knife for homogenous film thickness
- let the cream film dry for 1 hour at RT
- LAB is measured by Minolta Chroma Meter CR-300, 3 measuring spots on each card
- average L value is calculated and compared

The results are outlined in Table 1

**Table 1: Sunscreen formulations (O/W emulsions)**

| **INCI** | **Ref 1** | **Inv 1** | **Ref 2** | **Inv 2** | **Ref 3** | **Inv 3** |
|---|---|---|---|---|---|---|
| cetearyl alcohol | 2.00 | 2.00 | 2.00 | 2.00 | 2.00 | 2.00 |
| polyacrylamide; c13-14 isoparaffin; laureth-7 | 0.25 | 0.25 | 0.25 | 0.25 | 0.25 | 0.25 |
| ceteareth-25 | 2.00 | 2.00 | 2.00 | 2.00 | 2.00 | 2.00 |
| behenyl alcohol | 2.00 | 2.00 | 2.00 | 2.00 | 2.00 | 2.00 |
| C12-15 alkyl benzoate | 13.00 | 10.00 | 13.00 | 10.00 | 13.00 | 10.00 |
| phenoxyethanol; ethylhexylglycerin | 1.00 | 1.00 | 1.00 | 1.00 | 1.00 | 1.00 |
| phenethyl benzoate; | 5.00 | 5.00 | 5.00 | 5.00 | 5.00 | 5.00 |
| bis-ethylhexyloxyphenol methoxyphenyl triazine | - | 3.00 | - | 3.00 | - | 3.00 |
| ceteareth-6; stearyl alcohol | 2.00 | 2.00 | 2.00 | 2.00 | 2.00 | 2.00 |
| zinc oxide; triethoxycaprylylsilane (PARSOL^{®} ZX) | 5.00 | 5.00 | - | - | - | - |
| titanium dioxide; silica; dimethicone (PARSOL^{®} TX) | 5.00 | 5.00 | - | - | - | - |
| titanium dioxide; silica; dimethicone; (Parsol^{®} TX) | - | - | 5.00 | 5.00 | | |
| zinc oxide (Z-Cote^{®}) | - | - | - | - | 5.00 | 5.00 |
| titanium dioxide; silica (Eusolex^{®} T-AVO) | - | - | - | - | 5.00 | 5.00 |
| disodium edta | 0.20 | 0.20 | 0.20 | 0.20 | 0.20 | 0.20 |
| xanthan gum | 0.30 | 0.30 | 0.30 | 0.30 | 0.30 | 0.30 |
| butylene glycol | 3.00 | 3.00 | 3.00 | 3.00 | 3.00 | 3.00 |
| methylene bis-benzotriyzolyl tetramethylbutylphenol; aqua; decyl glucoside; propylene glycol; xanthan gum (Parsol^{®} MAX) | - | | 6.00 | 6.00 | - | - |
| aqua | Ad 100 | Ad 100 | Ad 100 | Ad 100 | Ad 100 | Ad 100 |
| **SPF (calculated*)** | **13.2** | **21.4** | **17.3** | **31.7** | **13.2** | **21.4** |
| **L value (average 9 spots)** | **39** | **36** | **43** | **37** | **42** | **38** |

| | | | | | | |
|---|---|---|---|---|---|---|
| *DSM sunscreen optimizer | | | | | | |

As can be retrieved from Table 1, the addition of BEMPT led to a reduction of the L value, reflecting a decreased whitening effect of the composition, which effect is particularly pronounced when an inorganic with an organic pigment is combined.

## Claims

1. A sunscreen composition containing as UV-filters solely a combination of the UV-filter substances i) bis ethylhexyloxyphenol methoxyphenyl triazine (BEMT) and (ii) two or more pigmentary UV-filters, wherein the two or more pigmentary UV-filters comprise at least two surface coated inorganic UV-filters or at least one surface coated inorganic UV-filter and at least one micronized insoluble organic UV-filter.

2. The sunscreen composition according to claim 1, wherein the amount of bis-ethylhexyloxyphenol methoxyphenyl triazine is selected in the range from 0.4 to 10 wt.-%, preferably from 0.8 to 5 wt.-%, most preferably from 1 to 5 wt.-%, based on the total weight of the composition.

3. The sunscreen composition according to claim 1 or 2, wherein the amount of the two or more pigmentary UV-filters is selected in the range from 0.5 to 25 wt.-%, preferably in the range from 1 to 20 wt.-%, most preferably in the range from 2 to 15 wt.-%, based on the total weight of the composition.

4. The sunscreen composition according to any one of claims 1 to 3, wherein the two or more pigmentary UV-filters comprise at least one titanium dioxide UV-filter and at least one zinc oxide UV-filter.

5. The sunscreen composition according to any one of claims 1 to 3, wherein the two or more pigmentary UV-filters comprise at least one inorganic UV-filter and at least micronized methylene bis-benzotriazolyl tetramethylbutylphenol.

6. The sunscreen composition according to any one of the preceding claims, wherein the inorganic UV-filters are selected from the group of double coated titanium dioxide having an inner silica and an outer dimethicone coating, silica coated titanium dioxide, and zinc oxide coated with triethoxycaprylylsilane.

7. The sunscreen compositions according to any one of the preceding claims, wherein the composition is an O/W emulsion comprising an oily phase dispersed in an aqueous phase in the presence of an O/W emulsifier, preferably in the presence of a cetyl phosphate emulsifier, most preferably in the presence of potassium cetyl phosphate.

8. The sunscreen composition according to any one of the preceding claims, wherein the composition further comprises one or more of butylene glycol, dicaprylate/dicaprate, phenethyl benzoate, C₁₂-C₁₅ alkyl benzoate, dibutyl adipate, diisopropyl sebacates, dicaprylyl carbonate, di-C₁₂-₁₃ alkyl tartrates, hydrogenated castor oil dimerates, triheptanoin, C₁₂-₁₃ alkyl lactates, C₁₆₋₁₇ alkyl benzoates, propylheptyl caprylates, caprylic/capric triglycerides, diethylhexyl 2,6-naphthalate, octyldodecanol, ethylhexyl cocoatesdibutyl adipate, preferably of butylene glycol, dicaprylyl carbonate, phenethyl benzoate, C₁₂-C₁₅ alkylbenzoate, caprylyl carbonate, capric/caprylic triglyceride, most preferably of butylene glycol, phenethyl benzoate and C₁₂-C₁₅ alkylbenzoate.

9. The sunscreen composition according to any one of the preceding claims, wherein the composition further comprises one or more of ethanol, phenoxyethanol and ethylhexylglycerin, preferably of phenoxyethanol and ethylhexylglycerin.

10. The sunscreen composition according to any one of the preceding claims, wherein the composition further comprises one or more of xanthan gum, crosslinked acrylate/C₁₀-C₃₀ alkyl acrylate polymer, hydroxyethyl acrylate/sodium acryloyldimethyl taurate copolymer, preferably xanthan gum.

11. The sunscreen composition according to any one of the preceding claims, wherein the composition further comprises one or more of behenyl alcohol, cetyl alcohol, cetearyl alcohol, stearyl alcohol and glyceryl stearate, preferably of behenyl alcohol, stearyl alcohol and cetearyl alcohol.

12. The sunscreen composition according to anyone of the preceding claims, wherein the composition has a SPF of at least 20.

13. Use of bis-ethylhexyloxyphenol methoxyphenyl triazine to reduce the whitening effect of two or more pigmentary UV-filters comprised in a sunscreen composition after application to the skin.

## Patentansprüche

1. Sonnenschutzzusammensetzung, die als UV-Filter ausschließlich eine Kombination der UV-Filterstoffe i) Bisethylhexyloxyphenolmethoxyphenyltriazin (BEMT) und (ii) zwei oder mehr pigmentäre UV-Filter enthält, wobei die zwei oder mehr pigmentären UV-Filter wenigstens zwei oberflächenbeschichtete anorganische UV-Filter oder wenigstens einen oberflächenbeschichteten anorganischen UV-Filter und wenigstens einen mikronisierten unlöslichen organischen UV-Filter umfassen.

2. Sonnenschutzzusammensetzung nach Anspruch 1, wobei die Menge von Bisethylhexyloxyphenolmethoxyphenyltriazin in dem Bereich von 0,4 bis 10 Gew.-%, vorzugsweise von 0,8 bis 5 Gew.-%, höchst bevorzugt von 1 bis 5 Gew.-%, bezogen auf das Gesamtgewicht der Zusammensetzung, ausgewählt ist.

3. Sonnenschutzzusammensetzung nach Anspruch 1 oder 2, wobei die Menge des einen oder der mehreren pigmentären UV-Filter in dem Bereich von 0,5 Gew.-% bis 25 Gew.-%, vorzugsweise in dem Bereich von 1 Gew.-% bis 20 Gew.-%, höchst bevorzugt in dem Bereich von 2 Gew.-% bis 15 Gew.-%, bezogen auf das Gesamtgewicht der Zusammensetzung, ausgewählt ist.

4. Sonnenschutzzusammensetzung nach einem der Ansprüche 1 bis 3, wobei die zwei oder mehr pigmentären UV-Filter wenigstens einen Titandioxid-UV-Filter und wenigstens einen Zinkoxid-UV-Filter umfassen.

5. Sonnenschutzzusammensetzung nach einem der Ansprüche 1 bis 3, wobei die zwei oder mehr pigmentären UV-Filter wenigstens einen anorganischen UV-Filter und wenigstens mikronisiertes Methylenbisbenzotriazolyltetramethylbutylphenol umfassen.

6. Sonnenschutzzusammensetzung nach einem der vorstehenden Ansprüche, wobei die anorganischen UV-Filter ausgewählt sind aus der Gruppe von doppelt beschichtetem Titandioxid mit einer inneren Siliciumdioxid- und einer äußeren Dimethiconbeschichtung, siliciumdioxidbeschichtetem Titandioxid und mit Triethoxycaprylsilan beschichtetem Zinkoxid.

7. Sonnenschutzzusammensetzung nach einem der vorstehenden Ansprüche, wobei die Zusammensetzung eine O/W-Emulsion umfassend eine ölige Phase dispergiert in einer wässrigen Phase in Gegenwart eines O/W-Emulgators, vorzugsweise in Gegenwart eines Cetylphosphat-Emulgators, höchst bevorzugt in Gegenwart von Kaliumcetylphosphat, ist.

8. Sonnenschutzzusammensetzung nach einem der vorstehenden Ansprüche, wobei die Zusammensetzung ferner eines oder mehrere von Butylenglycol, Dicaprylat/Dicaprat, Phenethylbenzoat, C₁₂-C₁₅-Alkylbenzoat, Dibutyladipat, Diisopropylsebacaten, Dicaprylylcarbonat, Di-C₁₂-₁₃-alkyltartraten, hydrierten Rizinusöldimeraten, Triheptanoin, C₁₂₋₁₃-Alkyllactaten, C₁₆₋₁₇-Alkylbenzoaten, Propylheptylcaprylaten, Capryl-/Caprintriglyceriden, Diethylhexyl-2,6-naphthalat, Octyldodecanol, Ethylhexylcocoatdibutyladipat, vorzugsweise von Butylenglycol, Dicaprylylcarbonat, Phenethylbenzoat, C₁₂-C₁₅-Alkylbenzoat, Caprylylcarbonat, Capryl-/Caprintriglycerid, höchst bevorzugt von Butylenglycol, Phenethylbenzoat und C₁₂-C₁₅-Alkylbenzoat umfasst.

9. Sonnenschutzzusammensetzung nach einem der vorstehenden Ansprüche, wobei die Zusammensetzung ferner eines oder mehrere von Ethanol, Phenoxyethanol und Ethylhexylglycerin, vorzugsweise Phenoxyethanol und Ethylhexylglycerin umfasst.

10. Sonnenschutzzusammensetzung nach einem der vorstehenden Ansprüche, wobei die Zusammensetzung ferner eines oder mehrere von Xanthangummi, vernetztem Acrylat/C₁₀-C₃₀-Alkylacrylat-Polymer, Hydroxyethylacrylat/Natriumacryloyldimethyltaurat-Copolymer, vorzugsweise Xanthangummi, umfasst.

11. Sonnenschutzzusammensetzung nach einem der vorstehenden Ansprüche, wobei die Zusammensetzung ferner eines oder mehrere von Behenylalkohol, Cetylalkohol, Cetearylalkohol, Stearylalkohol und Glycerylstearat, vorzugsweise von Behenylalkohol, Stearylalkohol und Cetearylalkohol, umfasst.

12. Sonnenschutzzusammensetzung nach einem der vorstehenden Ansprüche, wobei die Zusammensetzung einen SPF von wenigstens 20 aufweist.

13. Verwendung von Bisethylhexyloxyphenolmethoxyphenyltriazin zum Verringern der Aufhellungswirkung von zwei oder mehr pigmentären UV-Filtern, die in einer Sonnenschutzzusammensetzung enthalten sind, nach Aufbringen auf die Haut.

## Revendications

1. Composition d'écran solaire contenant comme filtres UV uniquement une combinaison des substances de filtre UV i) bis ethylhexyloxyphenol methoxyphenyl triazine (BEMT) et ii) deux ou plusieurs filtres UV pigmentaires, dans laquelle les deux ou plusieurs filtres UV pigmentaires comprennent au moins deux filtres UV inorganiques revêtus en surface ou au moins un filtre UV inorganique revêtu en surface et au moins un filtre UV organique insoluble micronisé.

2. Composition d'écran solaire selon la revendication 1, dans laquelle la quantité de bis-ethylhexyloxyphenol methoxyphenyl triazine est choisie dans la plage de 0,4 à 10 % en poids, préférablement de 0,8 à 5 % en poids, le plus préférablement de 1 à 5 % en poids, sur la base du poids total de la composition.

3. Composition d'écran solaire selon la revendication 1 ou 2, dans laquelle la quantité des deux ou plusieurs filtres UV pigmentaires est choisie dans la plage de 0,5 à 25 % en poids, préférablement dans la plage de 1 à 20 % en poids, le plus préférablement dans la plage de 2 à 15 % en poids, sur la base du poids total de la composition.

4. Composition d'écran solaire selon l'une quelconque des revendications 1 à 3, dans laquelle les deux ou plusieurs filtres UV pigmentaires comprennent au moins un filtre UV de dioxyde de titane et au moins un filtre UV d'oxyde de zinc.

5. Composition d'écran solaire selon l'une quelconque des revendications 1 à 3, dans laquelle les deux ou plusieurs filtres UV pigmentaires comprennent au moins un filtre UV inorganique et au moins du methylene bis-benzotriazolyl tetramethylbutylphenol micronisé.

6. Composition d'écran solaire selon l'une quelconque des revendications précédentes, dans laquelle les filtres UV inorganiques sont choisis dans le groupe du dioxyde de titane doublement revêtu ayant un revêtement interne de silice et un revêtement externe de diméthicone, du dioxyde de titane revêtu par une silice, et de l'oxyde de zinc revêtu par du triéthoxycaprylylsilane.

7. Compositions d'écran solaire selon l'une quelconque des revendications précédentes, dans lesquelles la composition est une émulsion H/E comprenant une phase huileuse dispersée dans une phase aqueuse en présence d'un émulsifiant H/E, préférablement en présence d'un émulsifiant de type cetyl phosphate, le plus préférablement en présence de potassium cetyl phosphate.

8. Composition d'écran solaire selon l'une quelconque des revendications précédentes, dans laquelle la composition comprend en outre l'un ou plusieurs parmi butylène glycol, dicaprylate/dicaprate, benzoate de phénéthyle, benzoate d'alkyle en C₁₂-C₁₅, adipate de dibutyle, sébaçates de diisopropyle, carbonate de dicaprylyle, tartrates de dialkyle en C₁₂-₁₃, dimérates d'huile de ricin hydrogénée, triheptanoïne, lactates d'alkyle en C₁₂-₁₃, benzoates d'alkyle en C₁₆-₁₇, caprylates de propylheptyle, triglycérides capryliques/capriques, 2,6-naphtalate de diéthylhexyle, octyldodécanol, cocoates d'éthylhexyle, adipate de dibutyle, préférablement parmi butylène glycol, carbonate de dicaprylyle, benzoate de phénéthyle, benzoate d'alkyle en C₁₂-C₁₅, carbonate de caprylyle, triglycéride caprique/caprylique, le plus préférablement parmi butylène glycol, benzoate de phénéthyle et benzoate d'alkyle en C₁₂-C₁₅.

9. Composition d'écran solaire selon l'une quelconque des revendications précédentes, dans laquelle la composition comprend en outre l'un ou plusieurs parmi l'éthanol, le phénoxyéthanol et l'éthylhexylglycérine, préférablement parmi le phénoxyéthanol et l'éthylhexylglycérine.

10. Composition d'écran solaire selon l'une quelconque des revendications précédentes, dans laquelle la composition comprend en outre l'un ou plusieurs parmi une gomme xanthane, un polymère d'acrylate/acrylate d'alkyle en C₁₀-C₃₀ réticulé, un copolymère d'acrylate d'hydroxyéthyle/acryloyldiméthyltaurate de sodium, préférablement une gomme xanthane.

11. Composition d'écran solaire selon l'une quelconque des revendications précédentes, dans laquelle la composition comprend en outre l'un ou plusieurs parmi l'alcool béhénylique, l'alcool cétylique, l'alcool cétéarylique, l'alcool stéarylique et le stéarate de glycéryle, préférablement parmi l'alcool béhénylique, l'alcool stéarylique et l'alcool cétéarylique.

12. Composition d'écran solaire selon l'une quelconque des revendications précédentes, dans laquelle la composition a un SPF d'au moins 20.

13. Utilisation de bis-ethylhexyloxyphenol methoxyphenyl triazine pour réduire l'effet blanchissant de deux ou plusieurs filtres UV pigmentaires compris dans une composition d'écran solaire après application sur la peau.
